# EUROPÄISCHE PATENTANMELDUNG

(11) **EP 0 561 152 A1**
(43) Veröffentlichungstag der Anmeldung: **22.09.1993**
(21) Anmeldenummer: 93102038.2
(22) Anmeldetag: 10.02.1993
(51) Int. Cl.: C09D 167/00

(54) **Überzugsmittel, deren Verwendung als Klarlacke und Verfahren zur Herstellung von Mehrschichtlackierungen**

(30) Priorität: 15.02.1992 DE 4204611
(71) Anmelder: Herberts Gesellschaft mit beschränkter Haftung, D-42285 Wuppertal (DE)
(72) Erfinder: Klostermann, Peter, Dr., W-5600 Wuppertal 2 (DE); Schönrock, Hans-Martin, W-5600 Wuppertal 2 (DE); Schröter, Klaus, W-5830 Schwelm (DE); Kutzner, Thomas, W-4322 Sprockhövel 2 (DE)
(74) Vertreter: Türk, Gille, Hrabal, Leifert

(57) **Zusammenfassung**

Überzugsmittel mit einer Viskosität von 100 - 1000 mPa.s, rotationsviskosimetrisch gemessen bei 70^{o}C und einem Schergefälle von 235 s⁻¹, enthaltend
45 - 85 Gew.% eines oder mehrerer Polyester, die verzweigt aufgebaut und im wesentlichen frei von aromatischen Struktureinheiten sind, mit einem zahlenmittleren Molekulargewicht von 350 - 3000 und einer Polydispersität kleiner als 3,5,
10 - 40 Gew.% eines oder mehrerer Vernetzer auf Basis von Aminoplastharzen und/oder blockierten Di- und/oder Polyisocyanaten,
0 - 20 Gew.% eines oder mehrerer Reaktivverdünner, sowie
0 - 10 Gew.% eines oder mehrerer organischer Lösungsmittel enthält.

Sie sind besonders als heißspritzbare Klarlacke für Mehrschichtlackierungen geeignet.

## Beschreibung

Die Erfindung betrifft Überzugsmittel auf der Basis von Polyesterharzen, Aminoplastharzen und/oder blockierten Di- und/oder Polyisocyanaten, die ein hochfestkörperreiches Einkomponenteneinbrennsystem darstellen. Sie sind besonders zur Verwendung als heißspritzbare Klarlacke zur Herstellung von Mehrschichtlackierungen geeignet.

Heutige Automobilserienlackierungen bestehen meist aus einer Klarlack/Basislack-Decklackierung, die auf eine elektrophoretisch grundierte und mit Füller beschichtete Karosse aufgebracht wird. Dabei werden Basislack und Klarlack bevorzugt naß-in-naß appliziert, das heißt der Basislack wird nach einer Ablüftzeit gegebenenfalls unter Erwärmen und nach anschließender Applikation eines Klarlacks gemeinsam mit diesem eingebrannt, wie z.B. in EP-A-0 038 127 und EP-A-0 402 772 beschrieben wird. In diesem Zusammenhang geeignete Klarlacke sind z.B. in den EP-A-0 038 127 und EP-A-0 184 761 beschrieben.

Sowohl im Falle der Verwendung von einkomponentigen (1K) als auch von zweikomponentigen (2K) Klarlacken ist der Lackiervorgang mit Emissionen von umweltschädlichen Lösemitteln verbunden. Im Rahmen der steigenden Umweltschutzbestrebungen ist die Lackindustrie und der Lackverbraucher bemüht, Lösemittelemissionen weitgehend zu vermeiden. Eine Möglichkeit ist der Einsatz von 2-Komponenten-Systemen. Im Falle von beispielsweise isocyanatvernetzenden 2K-Klarlacken z.B. nach DE-OS 33 22 037 oder DE-PS 36 00 425 ist ein Overspray-Recycling naturgemäß nicht möglich. Das ergibt als Folge eine erhöhte Abfallmenge.

Ein weiterer Weg zur Erreichung dieses Zieles ist der Einsatz einkomponentiger festkörperreicher Einbrennklarlacke bei der Herstellung von Mehrschichtlackierungen. Der Lösemittelgehalt solcher Lacksysteme liegt aber in der Regel bei mindestens 50 Gew.-%. Die US-PS 4 419 407 beschreibt sogar Klarlacke mit einem Harzfestkörper von bis zu 65 Gew.-%, die naß-in-naß auf abgelüftete Basecoats appliziert zur Herstellung einer Mehrschichtlackierung verwendet werden können. Die Lacke haben den Nachteil, daß ihre anwendungstechnischen Eigenschaften, sowie die Oberfläche des fertigen Überzugs ungünstiger sind als bei Lacken mit geringerem Festkörper.

Die DE-OS 22 53 300 beschreibt heißspritzbare lösemittelfreie oder lösemittelarme Lacksysteme auf Basis hochmolekularer Polyester mit Molekulargewichten von 1500 bis 5000, niedermolekularen Polyolen und von Melaminharzen. Insbesondere werden Alkydharze als Polyester beschrieben. Die Beispiele enthalten noch Lösemittelanteile in den Überzugsmitteln von mehr als 20 %. Diese dienen zur Verringerung der Viskosität.

Die DE-PS 26 29 930 beschreibt heißspritzbare, lösemittelfreie oder nur geringe Lösemittelanteile enthaltende Einbrennüberzugsmittel auf Basis linearer Polyesterharze mit Säurezahlen unter 15, bevorzugt unter 5 mg KOH/g und Methoxymethylaminharz als Vernetzer. Die Polyesterharze enthalten aromatische Struktureinheiten.

Sowohl in der DE-PS 22 53 300 als auch in der DE-PS 26 29 930 werden pigmentierte Überzugsmittel beschrieben. Außerdem sind diese Produkte nicht ausreichend stabil gegen ultraviolette Strahlen.

Aufgabe der vorliegenden Erfindung ist es, ein flüssiges, einkomponentiges hochfestkörperreiches Einbrennlacksystem zur Verfügung zu stellen, welches insbesondere zur Herstellung von Klarlacküberzügen geeignet ist, rezyklisierbar ist und es erlaubt, die Lösemittelemission gering zu halten.

Diese Aufgabe wird dadurch gelöst, daß ein Überzugsmittel für eine Mehrschichtlackierung zur Verfügung gestellt wird, das 45 - 85 Gew.-% eines oder mehrerer Polyesterharze (I) sowie 10 - 40 Gew.-% mindestens eines Aminoplastharzes und/oder blockierten Polyisocyanats als Vernetzer (II), jeweils bezogen auf das gesamte Überzugsmittel, enthält, wobei (I) verzweigt aufgebaut und im wesentlichen frei von aromatischen Struktureinheiten ist,
eine zahlenmittlere Molmasse (Mₙ) von 350 - 3000 und eine Polydispersität von maximal 3,5 besitzt,
sowie eine Viskosität von 100 - 1000 mPa.s, gemessen rotationsviskosimetrisch bei 70°C und einem Schergefälle von 235 s⁻¹, aufweist. Das erfindungsgemäße Überzugsmittel kann gegebenenfalls noch Lösungsmittel, Reaktivverdünner sowie weitere lacktechnische Additive enthalten. Es kann in der Wärme appliziert werden, gegebenenfalls kann es durch Eindrücken von superkritischen Lösemitteln in eine applikationsfertige Form gebracht werden.

Die erfindungsgemäß verwendeten Polyesterharze sind verzweigt aufgebaut und enthalten im wesentlichen keine aromatischen Struktureinheiten. Sie besitzen eine Untergrenze der Säurezahl von 12, bevorzugt von 14 mg KOH/g, die Obergrenze der Säurezahl liegt bei 25, bevorzugt bei 20 mg KOH/g, die Untergrenze der Hydroxylzahl beträgt 100, bevorzugt 135, besonders bevorzugt 180 mg KOH/g, die Obergrenze der Hydroxylzahl beträgt 300, bevorzugt 275, besonders bevorzugt 240 mg KOH/g. Durch Auswahl der Edukte und Art der Reaktionsführung bedingt besitzen die erfindungsgemäß verwendeten Polyesterharze zahlenmittlere Molekularmassen von 350 bis 3000, bevorzugt von 500 bis 1500 oder von 1000 bis 2000 (Bestimmung durch Gelpermeationschromatographie mit Polystyrolstandard). Die Polydispersität (M_{w}/Mₙ) der erfindungsgemäß verwendeten Polyesterharze soll einen Wert von 3,5 nicht überschreiten.

Die Herstellung der erfindungsgemäß verwendeten Polyesterharze erfolgt durch Polykondensation von aliphatischen und/oder cycloaliphatischen, gegebenenfalls ungesättigten, von aromatischen Struktureinheiten freien Mono- und/oder Dicarbonsäuren und/oder deren Anhydriden mit aliphatischen und/oder cycloaliphatischen Polyalkoholen und gegebenenfalls aliphatischen und/oder cycloaliphatischen Monoalkoholen. Dabei werden die Mengenanteile der einzelnen Reaktionskomponenten so ausgewählt, daß verzweigte Polyester mit OH-Funktionalitäten von gleich oder größer als 2,3 resultieren.Pro Äquivalent Carboxylgruppe, das sich bis zu 45 % aus Monocarbonsäuren ableiten kann, werden mehr als 0,3 Äquivalente OH-Gruppen, die sich von Polyolen mit mindestens drei OH-Gruppen im Molekül ableiten, eingesetzt. Es wird mit einem OH-Überschuß von mindestens 1,20 Äquivalenten OH pro Äquivalent Carboxylgruppe gearbeitet.

Es können auch zugleich hydroxyl- und carboxylgruppentragende Verbindungen zur Herstellung der erfindungsgemäß verwendeten Polyesterharze eingesetzt werden, soweit diese Verbindungen frei von aromatischen Struktureinheiten sind. Beispiele dafür sind Dihydroxymonocarbonsäuren, wie z.B. Dimethylolpropionsäure.

Beispiele für (cyclo)aliphatische gegebenenfalls ungesättigte Mono- und Dicarbonsäuren sind Sebacinsäure, Decandicarbonsäure, Dodecandicarbonsäure, Adipinsäure, Azelainsäure, Maleinsäure, Bernsteinsäure, Fumarsäure, Tetrahydrophthalsäure, 1,4-Cyclohexandicarbonsäure, 1,3-Cyclohexandicarbonsäure, Hexahydrophthalsäure, Methylhexahydrophthalsäure, Dimerfettsäuren, Monocarbonsäuren mit einer Kohlenstoffzahl von 6 bis 24, sowie Säureanhydride der Maleinsäure, der Hexahydrophthalsäure und der Bernsteinsäure.

Beispiele für (cyclo)aliphatische Mono- und Polyalkohole sind hydriertes Bisphenol A, Ethylenglykol, Diethylenglykol, Propandiol-1,3, Propandiol-1,2, 2-Methylpropandiol-1,3, Dipropylenglykol, Glyzerin, Pentaerythrit, Neopentylglykol, Trimethylolpropan, Trimethylylolethan, Butandiol, Pentandiol, Hexandiol, Decandiol, Hydroxypivalinsäureneopentylglykolester und Monoalkohole mit einer Kohlenstoffzahl von 6 bis 24.

Die Herstellungsverfahren für derartige Polyesterharze sind dem Fachmann bekannt und beispielsweise in Houben-Weyl "Methoden der Organischen Chemie", Makromolekulare Stoffe II, Band 14/2, Seiten 1 - 42, Georg Thieme Verlag, 1962, und in Ullmann, "Encyklopädie der technischen Chemie", 4. Auflage, Band 19, Seiten 61 - 88 beschrieben.

Bevorzugt werden die erfindungsgemäß verwendeten Polyesterharze nach dem dem Fachmann bekannten Schmelzverfahren bei Reaktionstemperaturen von etwa 150 bis etwa 250°C hergestellt.

Die zur Herstellung der erfindungsgemäßen, insbesondere als Klarlacke geeigneten Überzugsmittel verwendeten Polyesterharze werden in Mengenanteilen von 45 - 85 Gew.-%, bevorzugt von 55 - 70 Gew.-%, bezogen auf fertigen Lack, eingesetzt. Sie können auch als Gemisch mehrerer unterschiedlicher Polyesterharze eingesetzt werden, soweit sie nur den vorstehend genannten Definitionen gerecht werden.

Als Vernetzerkomponente (II) werden Aminoplastharze (IIa) und/oder blockierte Isocyanate (IIb) eingesetzt. Es handelt sich um teilweise oder vollständig veretherte Amin-Formaldehyd-Kondensationsharze und/oder blockierte Polyisocyanate mit mindestens zwei reaktiven Stellen pro Molekül.

Im Folgenden werden Beispiele für Verbindungen (IIa) angegeben: Amin-Formaldehyd-Kondensationsharze, die beispielsweise durch Reaktion von Aldehyden mit Melamin entstehen. Die Aldehyde können dabei monofunktionell, aber auch polyfunktionell sein. Beispiele hierfür sind Formaldehyd und seine Polymerisationsprodukte, wie Paraformaldehyd, Polyoxymethylen, Trioxan oder aliphatische und cyclische Aldehyde, wie Glyoxal, Acetaldehyd, Acrolein, Propionaldehyd, Butyraldehyd und Furfural. Je nach Reaktionsbedingungen (pH-Wert, Temperatur) und Methylolierungsgrad werden Harze mit verschiedenen Molmassen und unterschiedlicher Reaktivität erhalten. Die Kondensation mit Formaldehyd, Furfural, Paraformaldehyd, Polyoxymethylen oder Trioxan wird im allgemeinen unter Zusatz von schwachen Säuren oder Basen als Katalysator ausgeführt. Starke Säuren werden beispielsweise verwendet bei Kondensation mit Acrolein, Glyoxal, Acetaldehyd, Propionaldehyd oder Butyraldehyd. Hierbei wird das primäre Reaktionsprodukt neutralisiert, dann Aldehyd zugesetzt und unter Zusatz von schwachen Säuren oder Basen weiter reagiert. Der bevorzugte Aldehyd ist Formaldehyd. Die Alkoholgruppen, bevorzugt Methylolgruppen, der Aldehyd-Kondensationsprodukte werden teilweise oder bevorzugt vollständig mit Alkoholen verethert.

Es werden solche Amin-Formaldehydharze bevorzugt, deren Hauptmenge an Methylolgruppen mit Monoalkoholen oder deren Gemischen umgesetzt ist.

Besonders bevorzugt werden Methanol, Ethanol, Propanol, Butanol, Heptanol, Benzylalkohol und andere aromatische Alkohole, cyclische Alkohole sowie Ethoxyethanol oder Butoxyethanol. Sollen Alkohole mit mehr als 4 C-Atomen eingebaut werden, so wird die Methylolgruppe erst mit einem niedrigeren Alkohol verethert und anschließend der höhere Alkohol durch Umetherung eingeführt. Die bevorzugten Alkohole sind niedere aliphatische Monoalkohole, wie Methanol und/oder Butanol und seine Isomeren. Besonders bevorzugt werden Melaminharze, die mit 3 bis 6 Molen Formaldehyd umgesetzt und anschließend vollständig mit Methanol verethert sind. Die Harze werden nach dem Stand der Technik hergestellt und von vielen Firmen als Verkaufsprodukte angeboten. Bei Veretherung mit Hydroxyalkyl(meth)acrylaten oder Allylalkohol entstehen ungesättigte Melaminharztypen. Es können auch übliche carbamylmethylierte Melaminharze eingesetzt werden, die durch Reaktion von alkoxymethylierten Melaminharzen mit Alkylcarbamaten unter schwach sauren Bedingungen hergestellt werden.

Solche Kondensate vom Typ (IIa) sind beispielsweise beschrieben in Ullmann "Encyclopedia of Industrial Chemistry", 5. Auflage, Vol. A2, Kapitel "Aminoresins", Seiten 115 - 141 (1985) und Houben-Weyl, "Methoden der Organischen Chemie", Band 14/2, Seiten 319 - 388 (1962).

Eine Gruppe von Beispielen für Vernetzungsmittel vom Typ (IIb) stellt die Klasse der verkappten bzw. blockierten Polyisocyanate, die Diisocyanate einschließen , dar. Als verkappte Isocyanate können beliebige Di- und/oder Polyisocyanate verwendet werden, bei denen die Isocyanatgruppen mit einer Verbindung umgesetzt worden sind, die aktiven Wasserstoff enthält. Die verkappten Di- und/oder Polyisocyanate reagieren bei erhöhter Temperatur, in der Regel zwischen etwa 90 und 220°C mit den Filmbildnern. Blockierte Di- und/oder Polyisocyanate werden z.B. dadurch hergestellt, daß man ein multifunktionelles Isocyanat mindestens mit einer stöchiometrischen Menge an einer monofunktionellen, aktiven Wasserstoff (Zerewitinoff-Reaktion) enthaltenden Verbindung zweckmäßig bei Temperaturen von 50 bis 80°C umsetzt, wobei gegebenenfalls übliche Katalysatoren, wie basische Katalysatoren, wie tertiäre Amine und/oder geringe Mengen an Zinnsalzen, wie Dibutylzinndilaurat, zugegeben werden können. Als Di- und/oder Polyisocyanate können auch entsprechende isocyanatgruppenhaltige Prepolymere verwendet werden. Die organischen Di- und/oder Polyisocyanate weisen eine mittlere Molmasse von 112 bis 2000, bevorzugt 140 bis 1000, und zweckmäßig eine mittlere Isocyanatfunktionalität von 2 bis 8 auf. Geeignete Polyisocyanate sind beispielsweise Verbindungen der idealisierten Formel

E (N = C = O)ₛ

in welcher E für einen aliphatischen Kohlenwasserstoffrest mit 2 bis 18, vorzugsweise 6 bis 10 Kohlenstoffatomen, einen cyclischen Kohlenwasserstoffrest mit 6 bis 15 Kohlenstoffatomen und s für eine Zahl von 2 bis 5, vorzugsweise 2 bis 3, steht.

Typische Beispiele für derartige Polyisocyanate sind Propylendiisocyanat, Ethylethylendiisocyanat, Dimethylethylendiisocyanat, Trimethylendiisocyanat, Pentamethylendiisocyanat, Hexamethylendiisocyanat, Trimethylhexandiisocyanat, 1,12-Dodecandiisocyanat, 1,18-Octadecandiisocyanat, Cyclopentandiisocyanat, Cyclohexan-1,3- und 1,4-diisocyanat sowie beliebige Gemische dieser Isomeren, Methylcyclohexandiisocyanat, 1-Isocyanato-3,3,5-trimethyl-5-isocyanatomethylcyclohexan, Perhydro-2,4'- und/oder - 4,4'-diphenylmethandiisocyanat. Besonders bevorzugt werden großtechnisch erzeugte Diisocyanate wie Hexandiisocyanat, Isophorondiisocyanat oder Dicyclohexylmethan-diisocyanat. Außer den beispielhaft genannten, niedermolekularen Polyisocyanaten können auch die in der Polyurethanchemie bekannten höhermolekularen Isocyanat-Polymere auf Basis urethangruppenfreier Polyisocyanate und höhermolekularer Polyhydroxyverbindungen als Polyisocyanatkomponente eingesetzt werden. Zweckmäßig werden hierbei (n + 1) Mol der oben beschriebenen Diisocyanate mit n Mol einer gegenüber Isocyanat reaktiven difunktionellen Verbindung bei Temperaturen von zweckmäßig 50 bis 120°C in der Schmelze oder in Gegenwart inerter Lösemittel umgesetzt, die sowohl niedrigmolekular als auch hochmolekular mit einer Molmasse von 62 bis 1000, sein können. Arbeitet man mit einem Überschuß an Diisocyanat , so muß das überschüssige Isocyanat wieder abdestilliert werden. Als niedermolekulare Dialkohole werden zweckmäßig die verschiedenen Isomere von linearen, verzweigten und cyclischen Kohlenstoffverbindungen mit 2 bis 20 Kohlenstoffatomen und zwei sekundären und/oder primären Hydroxylgruppen verstanden. Typische Beispiele hierfür sind Butandiol-1,4, Hexandiol-1,6, Trimethylhexandiol, Bis(hydroxymethyl)cyclohexan, Neopentylglykol, Hydroxypivalinsäureneopentylglykolester, N-Methyl-diethanolamin. Geeignete höhermolekulare Polyhydroxylverbindungen sind auch die aus der Polyurethanchemie bekannten Polyesterdiole, Polycaprolactondiole, Polycaprolactamdiole, Polycarbonatdiole, Polyurethandiole und Polyglykoletherdiole. Verwendet werden können auch langkettige primäre und sekundäre Diamine, wie Hexandiamin-1,6, Addukte von 2 Mol Glycidylether oder Glycidylester an Hexandiamin, N,N'-Cyanethylethylendiamin oder Bis-N,N'-Cyanethylpolyoxypropylendiamin.

Als Polyisocyanate sind besonders gut die sogenannten "Lackpolyisocyanate" geeignet, die aus bekannten Diisocyanaten hergestellt werden. So entsteht aus Hexandiisocyanat und Wasser das Tris-(6-isocyanatohexyl)biuret. Durch Trimerisation von Hexandiisocyanat erhält man Tris-(6-isocyanatohexyl)isocyanurat, gegebenenfalls im Gemisch mit seinen höheren Homologen, sowie weiteren aus Isophorondiisocyanat aufgebauten Isocyanuratgruppen aufweisenden Polyisocyanaten. Sehr gut einsetzbare Isocyanate sind auch die Urethangruppen aufweisenden Polyisocyanate, die beispielsweise durch Umsetzung von überschüssigen Mengen an Diisocyanat mit einfachen, mehrwertigen Alkoholen der Molmasse 62 bis 300, insbesondere Trimethylolpropan und gegebenenfalls destillative Entfernung des nicht umgesetzten Diisocyanatüberschusses erhalten werden können. So werden beispielsweise blockierte Triisocyanate oder blockierte höhermolekulare Reaktionsprodukte von Triisocyanaten mit Dialkoholen besonders bevorzugt. Bei dieser Umsetzung werden zweckmäßig ungefähr folgende Molverhältnisse eingehalten: Triisocynat: Diol : Schutzgruppe wie y : (y-1) : (y+2), wobei y = 1 bis 6, bevorzugt 2 bis 3 ist. Mittel, die die Isocyanate blockieren, enthalten nur eine einzige Amin-, Amid-, Imid-, Lactam-, Thio- oder Hydroxylgruppe. Im allgemeinen werden flüchtige, aktiven Wasserstoff enthaltende Verbindungen mit niedrigen Molmassen, vorzugsweise von nicht mehr als 300, mehr bevorzugt von nicht mehr als 200, verwendet.

So haben sich beispielsweise bewährt: aliphatische oder cycloaliphatische Alkohole, wie n-Butanol, 2-Ethylhexanol, Cyclohexanol, Phenole, tertiär-Butylphenole, Dialkylaminoalkohole wie Dimethylaminoethanol, Oxime wie Methylethylketoxim, Lactame, wie ε-Caprolactam oder Pyrrolidon-2, Imide wie Phthalimid oder N-Hydroxy-maleinimid, Hydroxyalkylester, Malonsäure- oder Acetessigsäureester.

Es werden aber auch ß-Hydroxyglykole oder -glykolether und Glykolamide empfohlen. Oxime und Lactame sind als Verkappungsmittel von besonderem Interesse, weil die damit verkappten Polyisocyanate bei relativ niedrigen Temperaturen reagieren. Zur Blockierung können auch mehr als eine Art von Schutzgruppe, bevorzugt solche mit unterschiedlicher Reaktivität, verwendet werden. Es ist so beispielsweise möglich, ein Gemisch von zwei oder mehreren unterschiedlich blockierten Polyisocyanaten zu verwenden oder ein Polyisocyanat einzusetzen, das mit zwei oder mehreren unterschiedlichen Schutzgruppen blockiert ist.

Die erfindungsgemäß zur Herstellung der Überzugsmitel verwendeten Vernetzerkomponenten (II) sind vorzugsweise frei von aromatischen Struktureinheiten und werden in Mengenanteilen von 10 bis 40, bevorzugt unter 30 Gew.-%, bezogen auf fertiges Überzugsmittel, eingesetzt. Die Verbindungen des Typs (II) können allein oder im Gemisch eingesetzt werden.

Weiterhin kann das erfindungsgemäße Überzugsmittel flüssige, niedrigmolekulare, hydroxyfunktionelle Reaktivverdüner enthalten, das heißt Verbindungen, die beim Einbrennvorgang in den Lackfilm chemisch eingebaut werden, mit mindestens 2 Hydroxylgruppen pro Molekül und OH-Zahlen im Bereich von 400 bis 800 mg KOH/g. Geeignet sind z.B. Polyole, wie Polyetherpolyole, Polyesterpolyole, Polycarbonatpolyole und Polyurethanpolyole mit Molekularmassen von beispielsweise 150 bis 1000. Geeignet sind beispielsweise Handelsprodukte, wie durch Reaktion von Polyolen mit Caprolacton erhältliche Polycaprolactonpolyole, durch Reaktion von Oxiranverbindungen mit Polyolen und/oder Wasser erhältliche Polyetherpolyole wie z.B. Triethylenglykol, oder durch Reaktion von Polyaminen mit cyclischen Carbonaten erhältliche Polyurethanpolyole.

Die Reaktivverdünner werden in Mengenanteilen von 0 bis 20 Gew.-%, bezogen auf fertiges Überzugsmittel eingesetzt. Die Obergrenze liegt bevorzugt unter 15 Gew.-%, die Untergrenze beträgt bevorzugt 5, besonders bevorzugt 10 Gew.-%. Die Reaktivverdünner können allein oder im Gemisch eingesetzt werden.

Das erfindungsgemäße Überzugsmittel kann Lösemittel in Mengenanteilen bis zu 10, bevorzugt unter 5, besonders bevorzugt unter 3 Gew.-%, bezogen auf fertiges Überzugsmittel, enthalten. Unter Lösemittel werden in diesem Zusammenhang übliche in der Lackindustrie gebräuchliche Lösemittel verstanden, insbesondere solche, deren Siedepunkt bzw. -bereich 170°C nicht unterschreitet. Sie sind im Lacksystem inert, das heißt sie werden beim Einbrennvorgang nicht in den Lackfilm chemisch eingebunden. Beispiele sind Paraffine, z.B. mit C₁₁ - C₁₃; Aromaten, einzeln oder im Gemisch, mit z.B. C₁₂ - C₁₄; oder Ester, z.B. 2,2,4-Trimethyl-1,3-pentandiolmono-isobutyrat.

Das erfindungsgemäße Überzugsmittel kann gegebenenfalls geringe Mengen Vernetzungskatalysator enthalten. Dabei handelt es sich um handelsübliche saure Katalysatoren wie beispielsweise organische Sulfonsäuren, wie p-Toluolsulfonsäure, Dodecylbenzolsulfonsäure, Dinonylnaphthalinsulfonsäure. Die sauren Katalysatoren werden in geblockter Form eingesetzt. Beispiele sind Salze der Sulfonsäuren mit Aminen, wie Diisopropylamin, Morpholin, Pyridin, 2-Amino-2-methyl-1-propanol. Besonders bevorzugt werden die mit Epoxidverbindungen geblockten Sulfonsäuren verwendet, wie z.B. mit Versaticsäureglycidylester geblockte p-Toluolsulfonsäure. Sie werden in üblichen Mengen eingesetzt.

Weiterhin kann das erfindungsgemäße Überzugsmittel für Klarlacküberzugsmittel übliche lacktechnische Additive, wie beispielsweise Lichtschutzmittel, Verlaufsmittel, Weichmacher, transparente Pigmente und Füllstoffe, Netzmittel, Antischaummittel, viskositäts- bzw. rheologiebeeinflussende Mittel (z.B. substituierte Polyharnstoffe), enthalten.

Die Herstellung der erfindungsgemäßen Überzugsmittel erfolgt durch einfaches Vermischen der Komponenten (I) und (II), sowie gegebenenfalls anschließendes Zumischen von Reaktivverdünnern, Lösemitteln, Katalysatoren, Additiven. Dabei können in fester Form vorliegende Additive in vorhandenen flüssigen Bestandteilen gelöst werden oder sie werden durch Vermahlen nach bekannten Verfahren auf eine geeignete Teilchengröße gebracht.

Bevorzugt wird so gearbeitet, daß die Komponente (II) der Komponente (I) bei erhöhter Temperatur zugemischt wird. Die Temperatur wird dabei so gewählt, daß eine Reaktion zwischen den Komponenten (I) und (II) sicher vermieden wird. Die Temperaturen betragen unter 100°C, bevorzugt unter 80°C.

Die erfindungsgemäßen Überzugsmittel sind Einkomponenten-Systeme. Sie zeichnen sich durch eine hohe Lagerstabilität aus und können mehr als sechs Monate ohne wesentliche Viskositätsänderungen gelagert werden. Als Substrate sind metallische Substrate, Kunststoffe oder mit Überzügen vorbeschichtete Substrate geeignet.

Das Überzugsmittel wird durch Rollen, Walzen oder Spritzen aufgetragen, bevorzugt über Spritzapplikationsverfahren. Beispiele dafür sind Druckluftspritzen, Airless-Spritzen oder elektrostatisches Spritzen. Eine besondere Ausführungsform dieser Verfahren sind Heißspritztechniken, die gegebenenfalls mit superkritischen Lösemitteln unterstützt, angewendet werden können.

Bevorzugt werden Heißspritztechniken angewendet, wie z.B. Hot-Air-Heißspritzen. Dabei wird ein Temperaturbereich von 40 - 90°C, bevorzugt von 60 - 80°C, gewählt. Die Temperatur wird so gewählt, daß ein gutes Verspritzen und ein guter Verlauf sichergestellt wird. Weiterhin soll das Überzugsmittel auch bei mehrmaligem Verspritzen keinen chemischen Veränderungen unterliegen, um eine Rezyklisierung zu ermöglichen. Die thermische Belastung des Überzugsmittels kann auch durch Maßnahmen am Applikationsgerät gering gehalten werden. So kann z.B. das Erwärmen auf Applikationstemperatur erst in der Spritzpistole erfolgen. Weiterhin können schonende Heiztechniken, z.B. durch Heißluft, angewendet werden. Durch diese Maßnahmen kann eine Recyclingfähigkeit sichergestellt werden. Dabei wird frischem Überzugsmittel ein Anteil gegebenenfalls aufgearbeiteten Oversprays zugesetzt.

Die erfindungsgemäßen Überzugsmittel weisen insbesondere als Klarlacküberzugsmittel für das Heißspritzverfahren Viskositäten von 100 - 400 mPa.s, bevorzugt von 120 - 200 mPa.s gemessen rotationsviskosimetrisch bei 70°C und einem Schergefälle von 235 s⁻¹ auf. Das Zahlenmittel des Molekulargewichts (Mₙ) der dafür bevorzugten Bindemittel beträgt 500 bis 1500. Dabei ist die Polydispersität bevorzugt kleiner als 3.0.

Ein weiteres bevorzugtes Verfahren ist die Spritzapplikation unter Verwendung von superkritischen Flüssigkeiten nach EP-A-0 321 607. Dabei wird in das Überzugsmittel vor der Applikation eine Komponente, die im überkritischen Zustand viskositätserniedrigend wirkt, eingedrückt, wobei die Mischung in den superkritischen Zustand überführt wird, so daß dadurch auf eine geeignete Applikationsviskosität eingestellt werden kann. Ein bevorzugtes Beispiel für eine solche Komponente ist Kohlendioxid. Das Eindrücken und Verspritzen wird gegebenenfalls durch erhöhte Temperaturen unterstützt. Dabei ist die Temperatur oberhalb der kritischen Temperatur und der Druck oberhalb des kritischen Drucks einzustellen. Im Fall von Kohlendioxid wird beispielsweise bei 35 bis 70°C und bevorzugt bei 40 bis 60°C gearbeitet. Der Druck liegt bevorzugt bei 90 bis 140 bar. Beim angewendeten Spritzverfahren handelt es sich beispielsweise um eine Airless-Spritzapplikation.

Die Obergrenze an Kohlendioxid wird durch die Löslichkeitsgrenze des Systems festgelegt, es darf keine Phasentrennung auftreten. Sie ist abhängig von den gewählten Druck- und Temperatur-Bedingungen. Bevorzugt beträgt die eingesetzte Kohlendioxidmenge, bezogen auf die Gesamtlackmischung, 10 bis 50 Gew.-%.

Die Viskosität des reinen unverdünnten Überzugsmittels für dieses spezielle Anwendungsverfahren beträgt 100 bis 1000 mPa.s, rotationsviskosimetrisch gemessen bei 70°C und einem Schergefälle von 235 s⁻¹. Das Zahlenmittel des Molekulargewichts der Komponente I beträgt bevorzugt 1000 bis 2000.

Die superkritische Komponente (z.B. das Kohlendioxid) entweicht bei der Applikation, es ist eine direkte Recyclisierung des Oversprays möglich, da keine Anreicherung der überkritischen Komponente im Überzugsmittel erfolgt. Unter Recyclisierung ist bei den erfindungsgemäßen Überzugsmitteln zu verstehen, daß diese nach dem Auffangen des Oversprays gegebenenfalls durch chemische und physikalische Verfahren wieder aufgearbeitet werden können. Ebenso ist es als bevorzugte Ausführungsform möglich, daß das aufgefangene Lackmaterial, gegebenenfalls nach Filtrieren und Zufügen fehlender Bestandteile direkt wieder eingesetzt werden kann.

Bei der Applikation wird so vorgegangen, daß auf das schon mit weiteren Lackschichten versehene Substrat, z.B. Werkstücke aus Metall oder Kunststoff wie Automobilkarossen oder Teile davon, der Klarlacküberzug aus dem erfindungsgemäßen Überzugsmittel aufgetragen wird. Das Substrat kann dabei gegebenenfalls eine erhöhte Temperatur von bis zu 50°C besitzen. Das kann durch übliche Verfahrensmaßnahmen, wie z.B. Heißluft, IR-Strahler oder Ofenheizung erreicht werden oder das Substrat besitzt noch höhere Temperaturen aus vorhergehenden Beschichtungsvorgängen. Damit kann eine bessere Lackoberfläche erzielt werden.

Nach einer gegebenenfalls zwischengeschalteten Ruhephase, die dem Verlauf dient, wird das applizierte Überzugsmittel durch Erwärmen vernetzt. Dabei kann das Substrat zur Erzielung einer gleichmäßigen Oberfläche um Längs- oder Querachsen gedreht werden, wie z.B. in EP-A-0 278 482 beschrieben.

Die Einbrenntemperaturen liegen bei 130 bis 170°C, bevorzugt bei 140 bis 160°C. Die Schichtdicke des eingebrannten Films beträgt etwa 20 - 60 µm, bevorzugt 35 - 50 µm. Dabei entsteht ein vernetzter, harter, glänzender Lacküberzug. Eine bevorzugte Ausführungsform ist die Applikation des erfindungsgemäßen Überzugsmittels als Klarlacküberzug auf einen Basislack, bevorzugt einen wäßrigen Basislack. Dabei kann naß-in-naß gearbeitet werden, oder der Basislack wird vorher durch Erwärmen getrocknet. Dabei entsteht eine besonders gute Haftung der beiden Schichten.

Mit erfindungsgemäß als Klarlack formulierten Überzugsmitteln können beispielsweise Basislacke überlackiert werden, die übliche Basis- oder Decklackpigmente enthalten können, bevorzugt enthalten sie Effektpigmente, wie z.B. Metallic-Pigmente oder Iriodin-Pigmente. Als Bindemittelbasis des Basislacks werden bevorzugt Polyester-, Polyurethan- oder Acrylatharze eingesetzt. Diese Bindemittel können gegebenenfalls über Vernetzer (einzeln oder im Gemisch), z.B. Melamin- oder Isocyanatderivate, vernetzt werden. Beispiele für Basislacke, die mit dem erfindungsgemäßen Klarlack überlackiert werden können finden sich in DE-A-36 28 124, DE-A-37 15 254, DE-A-37 22 005, DE-A-39 13 001 und DE-A-40 11 633.

Die erfindungsgemäßen Überzugsmittel eignen sich besonders für Klarlacke, die bevorzugt auf dem Kraftfahrzeugsektor, jedoch auch auf anderen Gebieten eingesetzt werden können. Die Verwendung des erfindungsgemäßen Überzugsmittels in der Mehrschichtlackierung ist besonders für die Automobilserien-Lackierung geeignet, sie kann jedoch auch für andere Zwecke verwendet werden, wie z.B. für Haushaltsgeräte oder in der Möbelindustrie.

Klarlacküberzugsmittel aus den erfindungsgemäßen Überzugsmitteln ergeben bei der Herstellung von Mehrschichtlackierungen nur geringe Lösemittelemissionen. Das Overspray eignet sich für ein direktes Recycling.

### Beispiel 1:

In einer für die Polyestersynthese geeigneten Reaktionsapparatur wurden 278 g Neopentylglykol, 57 g Ethylenglykol, 134 g Trimethylolpropan, 197 g Adipinsäure, 324 g Hexahydrophthalsäureanhydrid und 1 g hypophosphorige Säure langsam aufgeschmolzen und innig vermischt. Nach Erreichen von 170°C setzte Polykondensation unter Wasserabspaltung ein, wobei die Temperatur langsam auf maximal 250^{o}C gesteigert wurde, so daß Wasser kontinuierlich abdestillierte, ohne daß die Kolonnenkopftemperatur 100^{o}C überschritt. Nach Erreichen einer Säurezahl von 16 mg KOH/g wurde rasch abgekühlt.

Man erhielt einen Polyester mit einer OH-Zahl von 229 mg KOH/g und einer zahlenmittleren Molekularmasse von 800 (gelpermeationschromatographisch, geeicht mit Polystyrolstandard). Die Polydispersität betrug 2,5.

Zu 751,5 g des erhaltenen Polyesters wurden nach Abkühlen auf 70^{o}C 225,5 g Hexamethoxymethylmelamin (Polymerisationsgrad: 1,5) gegeben, gut vermischt und nach Abkühlen auf 60^{o}C mit 23 g Texanol vermischt.

79,42 Teile der vorstehend genannten Harzmischung wurden unter Rühren nacheinander mit 5,83 Teilen Triethylenglykol, 5,83 Teilen eines handelsüblichen Polycaprolactontriols mit einer zahlenmittleren Molmasse von 300, 2,91 Teilen des Diesters aus Phthalsäure und 2-Ethylhexanol, 1,75 Teilen Versaticsäureglycidylester-geblockter p-Toluolsulfonsäure, 1,46 Teilen eines Gemischs handelsüblicher Verlaufsmittel auf Polysiloxanbasis, 0,78 Teilen eines handelsüblichen nichtsäurebindenden Lichtschutzmittels auf HALS-Basis, 1,55 Teilen eines handelsüblichen Lichtschutzmittels auf Benzotriazol-Basis und 0,47 Teilen eines handelsüblichen Netzmittels auf Basis eines niedermolekularen Acrylatharzes vermischt.

Man erhielt ein Klarlacküberzugsmittel mit einer Viskosität von 160 mPa.s, gemessen im Rotationsviskosimeter bei 70^{o}C und einem Schergefälle von 235 s⁻¹.

### Beispiel 2:

Analog zu Beispiel 1 wurden 368 g Neopentylglykol, 129 g Trimethylolpropan, 189 g Adipinsäure, 312 g Hexahydrophthalsäureanhydrid und 1 g hypophosphorige Säure polykondensiert, bis eine Säurezahl von 13,5 mg KOH/g erreicht war.
Der erhaltene Polyester hatte eine OH-Zahl von 200 mg KOH/g und eine zahlenmittlere Molekularmasse von 800 (gelpermeationschromatographisch, geeicht mit Polystyrolstandard) bei einer Polydispersität von 2,4.

Bei 70^{o}C wurden 752,1 g des Polyesters mit 225 g Hexamethoxymethylmelamin (Polymerisationsgrad: 1,5) und 22,9 g Texanol vermischt.

82,61 Teile dieser Harzmischung wurden mit 5,65 Teilen Triethylenglykol, 6 Teilen des in Beispiel 1 genannten Polycaprolactontriols, 1,76 Teilen der geblockten Sulfonsäure gemäß Beispiel 1, 2,42 Teilen einer Mischung handelsüblicher Verlaufsmittel auf Polysiloxanbasis und jeweils 0,78 Teilen der in Beispiel 1 genannten Lichtschutzmittel innig vermischt. Man erhielt ein Klarlacküberzugsmittel mit einer Viskosität von 175 mPa.s, gemessen im Rotationsviskosimeter bei 70^{o}C und einem Schergefälle von 235 s⁻¹.

### Beispiel 3:

Analog Beispiel 1 wurden 199 g Neopentylglykol, 119 g Ethylenglykol, 139 g Trimethylolpropan, 205 g Adipinsäure, 337 g Hexahydrophthalsäureanhydrid und 1 g hypophosphorige Säure polykondensiert, bis eine Säurezahl von 16 mg KOH/g erreicht war. Der erhaltene Polyester hatte eine OH-Zahl von 206 mg KOH/g und eine zahlenmittlere Molekularmasse von 800 (gelpermeationschromatographisch, Polystyrolstandard) bei einer Polydispersität von 2,4.

Bei 70^{o}C wurden 752,1 g des Polyesters mit 225 g Hexamethoxymethylmelamin (Polymerisationsgrad: 1,5) und 22,9 g Texanol vermischt.

81 Teile dieser Mischung wurden mit 6 Teilen Triethylenglykol, 6 Teilen des in Beispiel 1 genannten Polycaprolactontriols, 1,7 Teilen der in Beispiel 1 aufgeführten blockierten Sulfonsäure, 3 Teilen einer Mischung handelsüblicher Verlaufsmittel auf Polysiloxanbasis, jeweils 1 Teil der in Beispiel 1 genannten Lichtschutzmittel und 0,3 Teilen des in Beispiel 1 genannten Netzmittels unter Rühren innig vermischt.
Man erhielt ein Klarlacküberzugsmittel mit einer Viskosität von 146 mPa.s, gemessen im Rotationsviskosimeter bei 70^{o}C und einem Schergefälle von 235 s⁻¹.

Die in den Beispielen 1 bis 3 genannten Klarlacküberzugsmittel wurden wie folgt untersucht:
Mit in der Automobilserienlackierung verwendetem handelsüblichem KTL (18 µm) und handelsüblichem Füller (35 µm) vorbeschichtete Karosseriebleche wurden mit handelsüblichem wasserverdünnbarem Metallicbasislack in einer Trockenschichtdicke von 15 µm lackiert und 6 min bei 80^{o}C vorgetrocknet. Direkt anschließend wurden die in Beispiel 1 bis 3 genannten Klarlacküberzugsmittel durch Heißspritzauftrag bei 70^{o}C naß-in-naß appliziert und 30 min bei 150^{o}C Objekttemperatur eingebrannt.

In allen Fällen erhielt man harte, glänzende, gut haftende Mehrschichtlackierungen mit guter optischer Qualität. Die Vernetzung des Klarlackes wurde mit einem Methylethylketon-getränkten Wattebausch geprüft. Nach 100 Doppelhüben war die Klarlackoberfläche unverändert.

### Beispiel 4:

Beispiel 3 wurde wiederholt mit dem Unterschied, daß in das Klarlacküberzugsmittel im Verhältnis 100 : 45 Kohlendioxid (31 Gew.% CO₂, bezogen auf Gesamtzusammensetzung) eingedrückt und bei 55^{o}C und 125 bar im Airless-Spritzverfahren appliziert wurde. Nach 5 min Ablüften wurde 30 min bei 150^{o}C eingebrannt. Man erhielt ein gleichartiges Lackierergebnis wie in Beispiel 3 beschrieben.

### Beispiel 5:

Analog zu Beispiel 1 wurden 431,1 g Pentaerythrit, 450,8 g Hexahydrophthalsäureanhydrid, 616,6 g Isononansäure und 1,5 g hypophosphorige Säure polykondensiert, bis eine Säurezahl von 18 mg KOH/g erreicht war.

Der erhaltene Polyester hatte ein OH-Zahl von 137 mg KOH/g und ein zahlenmittleres Molekulargewicht von 1900 (gelpermeationschromatographisch, Polystyrolstandard) bei einer Polydispersität von 3,2.

Bei 70^{o}C wurden 750 g des Polyesters mit 227 g Hexamethoxymethylmelamin (Polymerisationsgrad 1,5) und 23 g Shellsol R (aromatischer Kohlenwasserstoff) vermischt.

77,6 Teile dieser Harzmischung wurden mit 7 Teilen Triethylenglykol, 6,5 Teilen des in Beispiel 1 beschriebenen Polycaprolactontriols, 1,5 Teilen des in Beispiel 1 genannten Phthalsäurediesters, 1,8 Teilen der geblockten Sulfonsäure gemäß Beispiel 1, 3 Teilen des Gemisches handelsüblicher Verlaufsmittel gemäß Beispiel 1, 0,5 Teilen des HALS-Lichtschutzmittels gemäß Beispiel 1, 1,6 Teilen des Benzotriazol-Lichtschutzmittels gemäß Beispiel 1 und 0,5 Teilen des Netzmittels gemäß Beispiel 1 vermischt.

Man erhielt ein Klarlacküberzugsmittel mit einer Viskosität von 490 mPas, gemessen im Rotationsviskosimeter bei 70^{o}C und einem Schergefälle von 235 s⁻¹.

Die Applikation des Klarlacküberzugsmittels erfolgte bei gleichartigem Lackierergebnis analog zu Beispiel 4 mit dem Unterschied, daß Klarlacküberzugsmittel und Kohlendioxid im Verhältnis 100 : 72 (42 Gew.% CO₂, bezogen auf Gesamtzusammensetzung) zur Anwendung kamen und bei 60^{o}C und 125 bar appliziert wurde.

In den vorstehenden Beispielen 1 bis 3 wurden jeweils 1,07 Äquivalente Diol und 0,43 Äquivalente Triol mit einem Äquivalent Dicarbonsäure umgesetzt. Im vorstehenden Beispiel 5 wurden 1,3 Äquivalente Tetrol mit 0,4 Äquivalenten Monocarbonsäure und 0,6 Äquivalenten Dicarbonsäure umgesetzt.

## Patentansprüche

1. Überzugsmittel, enthaltend ein oder mehrere Polyesterharze sowie ein oder mehrere Aminoplastharze und/oder blockierte Di- und/oder Polyisocyanate,
**dadurch gekennzeichnet**,
daß es eine Viskosität von 100 - 1000 mPa.s, rotationsviskosimetrisch gemessen bei 70^{o}C und einem Schergefälle von 235 s⁻¹, aufweist und
45 - 85 Gew.% eines oder mehrerer Polyester, die verzweigt aufgebaut und im wesentlichen frei von aromatischen Struktureinheiten sind, mit einem zahlenmittleren Molekulargewicht von 350 - 3000 und einer Polydispersität kleiner als 3,5,
10 - 40 Gew.% eines oder mehrerer Vernetzer auf Basis von Aminoplastharzen und/oder blockierten Di- und/oder Polyisocyanaten,
0 - 20 Gew.% eines oder mehrerer Reaktivverdünner, und
0 - 10 Gew.% eines oder mehrerer organischer Lösungsmittel enthält.

2. Überzugsmittel nach Anspruch 1, dadurch gekennzeichnet, daß die OH-Zahl der Polyester bei 100 - 300 mg KOH/g liegt.

3. Überzugsmittel nach Anspruch 2, dadurch gekennzeichnet, daß die OH-Zahl 135 - 275 mg KOH/g beträgt.

4. Überzugsmittel nach einem der Ansprüche 1 bis 3, dadurch gekennzeichnet, daß die Säurezahl der Polyester bei 12 - 25 mg KOH/g liegt.

5. Überzugsmittel nach Anspruch 4, dadurch gekennzeichnet, daß die Säurezahl 14 - 20 mg KOH/g beträgt.

6. Überzugsmittel nach einem der Ansprüche 1 bis 5, dadurch gekennzeichnet, daß die Polydispersität (M_{w}/Mₙ) kleiner als 3,0 ist.

7. Überzugsmittel nach einem der Ansprüche 1 bis 6, dadurch gekennzeichnet, daß das Zahlenmittel des Molekulargewichts der Polyester 500 - 1500 beträgt.

8. Überzugsmittel nach einem der Ansprüche 1 bis 6, dadurch gekennzeichnet, daß das Zahlenmittel des Molekulargewichts der Polyester 1000 - 2000 beträgt.

9. Überzugsmittel nach einem der Ansprüche 1 bis 8, dadurch gekennzeichnet, daß es 10 - 30 Gew.% eines oder mehrerer Vernetzer enthält.

10. Überzugsmittel nach einem der Ansprüche 1 bis 9, dadurch gekennzeichnet, daß es als Vernetzer eines oder mehrere Melaminharze enthält.

11. Überzugsmittel nach einem der Ansprüche 1 bis 10, dadurch gekennzeichnet, daß es ein oder mehrere transparente Pigmente enthält.

12. Überzugsmittel nach einem der Ansprüche 1 bis 11, dadurch gekennzeichnet, daß es weniger als 3 Gew.% organisches Lösungsmittel enthält.

13. Überzugsmittel nach einem der Ansprüche 1 bis 12, dadurch gekennzeichnet, daß das Lösungsmittel einen Siedepunkt über 170^{o}C besitzt.

14. Überzugsmittel nach einem der Ansprüche 1 bis 13, dadurch gekennzeichnet, daß als Reaktivverdünner Polyole mit mindestens 2 OH-Gruppen pro Molekül, einer OH-Zahl im Bereich von 400 bis 800 und einer Molmasse von 150 bis 1000 enthalten sind.

15. Überzugsmittel nach einem der Ansprüche 1 bis 14, dadurch gekennzeichnet, daß das Überzugsmittel zwischen 5 und 15 Gew.%, bezogen auf das gesamte Überzugsmittel, eines oder mehrerer Reaktivverdünner enthält.

16. Überzugsmittel nach einem der vorhergehenden Ansprüche, dadurch gekennzeichnet, daß es zusätzlich ein oder mehrere superkritische Lösemittel enthält.

17. Verfahren zur Herstellung einer Mehrschichtlackierung, dadurch gekennzeichnet, daß auf eine Basislackschicht ein heißspritzbarer Klarlack aus einem Überzugsmittel gemäß einem der Ansprüche 1 bis 16 aufgetragen wird.

18. Verfahren nach Anspruch 17, dadurch gekennzeichnet, daß bei einer Spritztemperatur von 40 bis 90^{o}C gearbeitet wird.

19. Verfahren nach Anspruch 17, dadurch gekennzeichnet, daß in das Überzugsmittel vor der Applikation ein superkritisches Lösemittel eingedrückt wird.

20. Verfahren nach Anspruch 19, dadurch gekennzeichnet, daß superkritisches CO₂ eingedrückt wird.

21. Verfahren nach Anspruch 17, dadurch gekennzeichnet, daß bei einer Temperatur von 60 bis 80^{o}C gearbeitet wird.

22. Verfahren nach Anspruch 19 oder 20, dadurch gekennzeichnet, daß bei einer Temperatur von 35 bis 70^{o}C gearbeitet wird.

23. Verfahren nach einem der Ansprüche 17 bis 22, dadurch gekennzeichnet, daß das Substrat vor dem Beschichten eine erhöhte Temperatur aufweist.

24. Verfahren nach einem der Ansprüche 17 bis 23, dadurch gekennzeichnet, daß die Basislackschicht aus einem wäßrigen Überzugsmittel hergestellt wurde.

25. Verfahren nach einem der Ansprüche 17 bis 24, dadurch gekennzeichnet, daß naß-in-naß gearbeitet wird.

26. Verwendung der Überzugsmittel nach einem der Ansprüche 1 bis 16 zur Herstellung von Klarlacküberzügen.

27. Verwendung der Überzugsmittel nach einem der Ansprüche 1 bis 16 als heißspritzbare Klarlacke.

28. Substrat beschichtet mit einer Mehrschichtlackierung, erhalten nach dem Verfahren eines der Ansprüche 17 bis 25.
